# EUROPEAN PATENT SPECIFICATION

(11) **EP 3 223 759 B1**
(45) Date of publication and mention of the grant of the patent: **06.02.2019**
(21) Application number: 14805782.1
(22) Date of filing: 23.09.2014
(51) Int. Cl.: A61F 6/00

(54) **PACKING FOR A CONDOM**
VERPACKUNG FÜR EIN KONDOM
EMBALLAGE POUR PRÉSERVATIF

(30) Priority: 28.08.2014 EA 201400848
(43) Date of publication of application: 04.10.2017
(73) Proprietor: Obschestvo s ogranichennoy otvetstvennostyu "D3 PROEKT", St. Petersburg 190031 (RU)
(72) Inventor: Lepilin, Maksim Evgen'evich, St. Petersburg 194354 (RU)
(74) Representative: Williams, Paul Edwin
(86) International application number: PCT/EA2014/000018
(87) International publication number: WO 2016/029921

(56) References cited:
- WO-A1-90/08522
- WO-A1-2014/102584
- WO-A2-2005/107663
- WO-A2-2007/021184

## Description

### TECHNICAL FIELD

The present invention relates to a device of packing for barrier contraceptives, including condoms.

### BACKGROUND

Traditional packing for a condom represents a reliable covering made of flexible sheet material designed for airtight condom storage. Besides, it prevents early leakage of lubricant from the condom covering. For convenient usage such packings are provided with means to open the covering, for example a groove, a site with lower reliability on the edge of the packing, or an opening tape with one end left out to break the covering.

A deficiency of the traditional packing for a condom is a long and inconvenient packing opening process. This is due to the fact that before using the condom one has to waste time looking for a covering opening tool provided with the packing, for example, the groove, and next, having taken the packing edges on both sides with both hands, pulling it apart to make a tear. Under the circumstances when a condom is about to be used, extra seconds spent on the movements described, may be critical for a person. Herein, there is a chance to damage a condom in case of careless and hasty opening of the packing, because a person in tense emotional state may apply unnecessary extra force to tear the covering. Besides, when using a condom as designed, the contact between fingers and the condom itself is inevitable, thus compromising the hygiene required. Furthermore, the production process of such packing is rather time-consuming and complicated, as it is not only complicated to arrange the means of packing opening accurately, in the required place, but to provide airtightness of such packing is complicated either.

A packing for a condom is known (Patent Application WO9846495, published on October 22nd 1998) that comprises a covering wherein a condom is placed, the covering with an opening line at the center, throughout the entire surface, and flat grippers attached to the covering edges designed to rest the fingers when opening the covering. When using the packing known the flat grippers are to be pulled apart in a single plane. Herein, the covering is torn apart by the opening line to be split into two parts with a condom remaining in one part of the packing.

A deficiency of the known packing for a condom is the inevitable contact between hand fingers and the condom during its usage, because after opening the packing the condom must be extracted by hands. This compromises the hygiene required. Besides, in such packing it is complicated to provide airtightness between the covering and side grippers, particularly to prevent early leakage of hygienic lubricant, the condom is provided with in such a small amount, that the tiniest leakage thereof through the covering connection, in particular with flat gripper material results in malfunctioning thereof. This results from the fact, that parts of covering are connected to each other via foreign body. Furthermore, this makes the production process of such packing more complicated.

Packing for a condom is known (Patent EP1377242B1, published on May 24th 2006). This packing consists of a covering. A condom is located inside on four holders disposed at the corners of the covering, and fixing the condom therein. The holders are connected to two side grippers, placed outside the covering. The covering comprises an opening line, located by its central crosswise perimeter, parallel to side grippers. As the result, when opening via this line, the covering divides into two equal parts. The upper surface of each side gripper is ridged. The ridging of side grippers is left outside the covering and tight connection of the covering is placed on the surface of the grippers in front of the ridging described.

The known packing is opened via pressing on the side grippers with fingers resulting in tearing the packing apart through the opening line. Then the side grippers are stretched in different directions, and the packing is separated into two equal parts, containing a condom. Next, via catching the side grippers with fingers, the condom is put on the erect penis and removed from the packing by disengaging it from the hook-shaped holders with corresponding parts of the covering.

A deficiency of this packing for a condom is its technological complicacy resulting from airtightness issues in points where flat grippers protrude from the covering. This is due to the fact that in those points where grippers protrude from the covering, there are junction points, i.e. via sealing the covering onto the surface of the side grippers. It is thus complicated to achieve reliable sealing of the covering resulting in overall deterioration of the condom application process as the packing hygiene may have been tampered.

WO 2007/021184 discloses a condom applicator which comprises at least one arcuate guiding member for engaging on the outside surface of a condom, which in use extends adjacent a rolled up part of said condom along at least a part of the circumference of the condom. On at least one end of said guiding member a receiving loop is provided for receiving the rolled up part of the condom. The receiving loop is open on the underside and extends in use over the rolled up part of the condom.

WO 2005/107663 discloses a condom applicator which comprises a base with a split line across it and two flaps which are connected by flimsy hinges to the base. The base includes two generally parallel walls and a base which bound a trough which receives the hem ring of a condom. There is an opening in the base which is encircled by the inner wall and the teat of the condom lies across the opening.

EP 2 810 626 discloses a device of packing for barrier contraceptives such as condoms. The packing for a condom consists of the covering with the folded condom inside. The covering is connected to two side grippers, wherein each side gripper with its one end is placed inside the covering to be connected to, at least, one hook-shaped holder located inside the covering that is designed to fix the condom within the covering. The covering comprises the opening line that is located by the central crosswise perimeter of the covering, that, when opening the covering, splits the covering in two parts. The surface of the covering is provided with at least two enforcement ribs located parallel and symmetrical about the opening line that function as pressure concentrators while opening the covering by breaking it up. Each side gripper surface may be ridged by ruts to increase adhesion thus reducing the probability of sliding the fingers off when opening the packing.

EP 2 810 626 does not disclose four hook-shaped holders - one in each of four corners, and that the side grippers are entirely placed into the covering, the covering having a perimeter whose edges are sealed to each other airtight throughout the entire perimeter of the covering.

### SUMMARY OF THE INVENTION

The object of the invention is creation of new packing for condoms to simplify production technology and improve airtightness via preventing occasional lubricant leakage therefrom, thus maintaining hygiene when using the condom.

The object formulated gets solved by the packing for a condom according to claim 1.

In an embodiment, the opening line is made straight or zigzagged, preferably by a method which comprises perforating its contour or processing it with a laser beam.

Such new technical solution, with all essential features thereof, allows achieving the following technical result, namely: the airtightness of packing is improved with its production technology simplified. The result is achieved due to the fact that the packing airtight encloses holders with the condom located thereon, with other ends of side grippers that are opposite to the ends connected to the holders, to firmly fix its position set on holders. It allows the claimed packing production technology to be substantially simplified. Herein, as the covering edges are sealed airtight to each other throughout the entire perimeter of the covering, with its external contour outlined within the plane where the side grippers with holders are located to exclude the connection of the covering edges by a third body, which is often a foreign body, especially the free ends of the flat grippers, that in the prototype passed out from beneath the covering, thus guiding the connection of corresponding covering edges, so the airtightness of the packing is now improved. Besides, it is the technical solution claimed, that provides uniform tension of the covering throughout the entire surface of holders with the condom and grippers and that is achieved due to the fact that throughout the entire perimeter of the covering, with its external contour outlined, within the plane where grippers with holders are located, the covering edges are connected to each other, for example, by sealing.

The packing for a condom may be of various geometric shape, such as oval or rhombic, or any other shape convenient to be used with both hands.

Thus, a device of packing for a condom claimed allows keeping familiar visual perception of packing and the way of using it. Furthermore, it allows automating the process of the packing production, since traditional shape may be used. The covering itself may be made of two parts by covering one part with another part with a condom onto holders with grippers placed within.

Examples of particular embodiments of the subject matter as well as the practical applicability thereof are disclosed herein with reference to the following description and the drawings.
Fig.1 shows lateral view of the packing for a condom, where:
   - 1: represents the covering;
   - 2: represents a hook-shaped holder,
   - 3: represents the condom;
   - 4: represents a side gripper;
   - 5: represents covering edges;
   - 6: represents an opening line;
   - 7: represent a finger rest;
   - 8: represents ridging.
Fig.2 shows the upper view of the opened packing for a condom (zigzagged opening line).

### DETAILED DESCRIPTION OF THE INVENTION

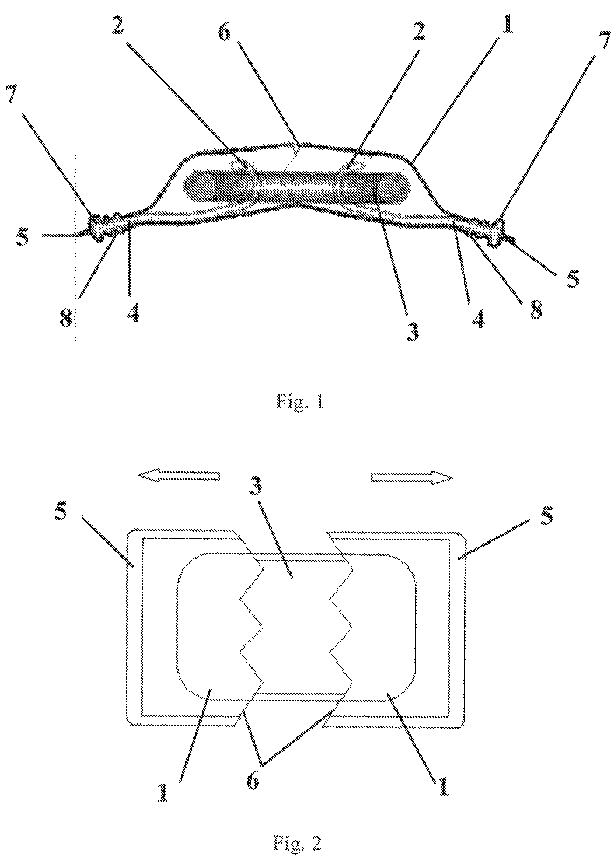

The packing for a condom claimed contains the covering 1 (Fig.1, 2). Located inside it, in its four corners, on four hook-shaped holders 2, is a condom 3 in a fixed position. The covering 1 envelops side grippers 4. Those are connected to the sides of hook-shaped holders 2. Herein, the side grippers 4 are entirely placed into the covering 1. The edges 5 of the covering 1 are airtight connected to each other throughout the entire perimeter of the covering 1, with its external contour outlined within the plane where side grippers 4 with holders 2 are located. Herein, the covering 1 has opening line 6 that embraces it by the center of the condom 3 to be placed parallel to side grippers 4 capable, when opening the covering 1 by this line, to divide the covering 1 into two parts (Fig.2). Herein, the covering is made of materials allowing tactile action by hand fingers (not shown by the drawing) onto the contours of free side grippers 4 ends and/or the part of the covering edges 5 of the covering 1 over side grippers 4 repeats the pattern, the pattern that was preliminarily put onto the surface of free of holders 2 ends of the side grippers 4. Herein, the material of the covering may be medical foil or corresponding polymer material.

Those skilled in the art would appreciate the opening line 6 may be made either straight or zigzagging via perforating its contour or laser treatment.

Side grippers may be provided with finger rests 7 to prevent hand fingers slipping when using the packing. Besides, the covering 1 with side grippers 4 may in cross section have a shape of an oval (not shown on the drawing), in longitudinal section it may have convex-concave profile (not shown on the drawing) that gradually narrows towards side grippers 4 ends. Implementing the packing shape in such a way facilitates faster opening of the covering 1, via reducing the efforts to be applied while breaking by the opening line 6.

The surface of each side gripper 4, throughout its portion that is free of holder 2, may have ridging 8, due to grooves (shown conventionally on the drawing) to improve adhesion and reduce the chance for finger slipping, when opening the packing, the grooves that an individual may feel with fingers through the covering 1, the feature that may be essential, for partially sighted.

The covering 1 may be made, for example, of two layers (the upper and the lower) of a polymer material, or hygienic foil, and comprise additional lubricant (not shown on the drawing) to preserve intactness and marketable appearance of the condom 3. Herein, the covering 1 is made via airtight gluing or sealing the edges of the two layers of the polymer material (shown conventionally on the drawing) throughout the entire contour within the location plane that is shared by grippers 4 with holders 2 and the condom thereon.

The invention claimed is to be used as follows.

To use the condom 3 as designed, the condom packing needs to be opened. To do so, one has to take, with both hands, the side grippers 4 and pull then up or down (in vertical plane), resulting in breaking the covering 1 by its opening line 6. Next, to fully divide the covering 1 into two parts, one has to pull side grippers 4 aside (in horizontal plane). Herein, due to the fixation of the condom 3 with hook-shaped holders 2, that the side grippers 4 are equipped with, the condom 3 is extended to increase the diameter of the condom 3 ring. It allows, without touching the condom 3 with fingers, putting it onto the erect penis (not shown on the drawing) fast and convenient, without touching the condom 3 with fingers. Upon the complete unwinding of the condom 3 ring, the hook-shaped holders 2 can be easily detached from the condom 3.

The opened condom packing is to be disposed of.

Thus, the present invention provides for the following technical results: production technology is simplified, as the covering now airtight envelops the holder with the condom thereon, with side grippers; packing usability is improved, as it makes possible to property orientate the item when used in practice with no need for visual control thus providing quick and convenient extraction of a condom still preserving its hygiene.

## Claims

1. Packing for a condom comprising a covering (1), placed inside which are hook-shaped holders (2), one in each of four corners, with a condom (3) in fixed position within, and side grippers (4) connected to the holders, with an opening line (6) implemented onto the packing, the opening line extending over a central part of the condom from and to positions substantially midway between pairs of the side grippers, and capable to divide the covering into two parts when opening the covering by this line, wherein the side grippers are entirely placed into the covering, the covering having a perimeter (5) whose edges are sealed to each other airtight throughout the entire perimeter of the covering, with its external contour outlined within a plane where side grippers with holders are located, the covering is made of material that allows tactile action, by hand fingers, on contours of side grippers ends that are distal from the hook-shaped holders, and/or the part of the covering edges over side grippers repeats a pattern defined in the surface of said distal ends of the side grippers.

2. Packing for a condom as claimed in claim 1, **characterized in that**, the opening line is straight or zigzagged.

3. A method of manufacturing packing for a condom as claimed in claim 1 or 2, **characterized in that**, the opening line is made straight or zigzagged via perforating its contour or processing it with a laser beam.

## Patentansprüche

1. Verpackung für ein Kondom, umfassend eine innenliegende Hülle (1), in der hakenförmige Halterungen (2) sind, eine in jeder der vier Ecken, mit einem Kondom (3) in einer festen Position darin, und Seitengreifer (4), die mit den Halterungen verbunden sind, mit einer Öffnungslinie (6), die an der Verpackung angebracht ist, die Öffnungslinie erstreckt sich über einen mittleren Teil des Kondoms von und zu Positionen, die im Wesentlichen auf halber Strecke zwischen den Paaren der Seitengreifer liegen, und in der Lage sind, die Hülle in zwei Teile zu teilen, wenn die Hülle an dieser Linie geöffnet wird, wobei
die Seitengreifer vollständig in der Hülle untergebracht sind, die Hülle hat einen Umfang (5), dessen Ränder im gesamten Umfang der Hülle vollständig luftdicht zueinander abgeschlossen sind, wobei ihre äußere Kontur innerhalb einer Fläche umrissen ist, auf der die Seitengreifer mit den Halterungen positioniert sind, die Hülle ist aus Material hergestellt, das einen taktilen Vorgang mit den Fingern an den Konturen der Seitengreifer-Enden erlaubt, die sich distal zu den hakenförmigen Halterungen befinden, und/oder der Teil der Hüllenränder über den Seitengreifern wiederholt ein Muster, das an der Oberfläche des besagten distalen Endes der Seitengreifer definiert ist.

2. Verpackung für ein Kondom nach Anspruch 1, **dadurch gekennzeichnet, dass** die Öffnungslinie gerade oder im Zickzack verläuft.

3. Ein Verfahren für die Herstellung einer Verpackung für ein Kondom nach Anspruch 1 oder 2, **gekennzeichnet dadurch, dass** die Öffnungslinie gerade oder im Zickzack durch Perforieren der Kontur oder Bearbeiten durch einen Laserstrahl hergestellt wird.

## Revendications

1. Emballage destiné à un préservatif comprenant une enveloppe (1), des supports en forme de crochet sont placés à l'intérieur (2), un dans chacun des quatre coins, avec un préservatif (3) en position fixe à l'intérieur et des pinces latérales (4) reliées aux supports, comportant une ligne d'ouverture (6) mise en oeuvre dans l'emballage, la ligne d'ouverture s'étendant sur une partie centrale du préservatif depuis et vers des positions pratiquement à mi-chemin entre les pinces latérales et apte à diviser l'enveloppe en deux parties, lors de l'ouverture de l'enveloppe par cette ligne, dans lequel
les pinces latérales sont entièrement placées dans l'enveloppe, l'enveloppe présentant un périmètre (5) dont les bords sont scellés l'un à l'autre hermétiquement à l'air sur toute l'étendue du périmètre de l'enveloppe, avec son contour externe tracé à l'intérieur d'un plan dans lequel se trouvent les pinces latérales comportant les supports, l'enveloppe est constituée d'un matériau qui permet une action tactile, par les doigts de la main, sur les contours des pinces latérales qui sont distales par rapport aux supports en forme de crochet et/ou la partie des bords de l'enveloppe sur les pinces latérales répète un motif défini dans la surface desdites extrémités distales des pinces latérales.

2. Emballage destiné à un préservatif selon la revendication 1, **caractérisé en ce que** la ligne d'ouverture est droite ou en zigzags.

3. Procédé de fabrication de l'emballage destiné à un préservatif selon la revendication 1 ou 2, **caractérisé en ce que** la ligne d'ouverture est faite droite ou en zigzags par l'intermédiaire de perforation de son contour ou de traitement avec un faisceau laser.
